# EUROPÄISCHE PATENTANMELDUNG

(11) **EP 1 336 392 A1**
(43) Veröffentlichungstag der Anmeldung: **20.08.2003**
(21) Anmeldenummer: 02003467.4
(22) Anmeldetag: 14.02.2002
(51) Int. Cl.: A61F 2/06

(54) **Gefässstütze und Kathetersystem**

(71) Anmelder: Geis, John S., 82031 Grünwald (DE); Braun, Michael, 71522 Backnang (DE)
(72) Erfinder: Geis, John S., 82031 Grünwald (DE); Braun, Michael, 71522 Backnang (DE)
(74) Vertreter: Cullinane, Marietta Bettina

(57) **Zusammenfassung**

Die Erfindung betrifft eine Gefäßstütze (1), die im wesentlichen zylindrischen Schlauch (12) aus flexiblem Material, der über seine Länge mindestens zwei aneinander angrenzende Bereiche (2, 3) aufweist, und mindestens ein Federelement (11, 11', 11"), das im entspannten Zustand den Schlauch (12) zumindest in dem Bereich (3) in dem dieses vorgesehen ist, in einem geöffneten Zustand hält und das ein radiales Zusammendrücken des Schlauches (12) zulässt, umfasst, dadurch gekennzeichnet, dass das mindestens eine Federelement (11, 11', 11") in einem der Bereiche (3) des Schlauches vorgesehen ist und der an diesen Bereich (3) angrenzende andere Bereich (2) frei von Federelementen (11, 11', 11") ist, wobei der Bereich (2), in dem kein Federelement (11, 11', 11") vorgesehen ist, sich bis zu einem der axialen Enden (121) des Schlauches (12) erstreckt. Weiterhin betrifft die Erfindung ein Kathetersystem zu Einführen einer solchen Gefäßstütze.

## Beschreibung

Die vorliegende Erfindung betrifft eine Gefäßstütze, insbesondere einen Stent, der bevorzugt als thorokale Aorten-Stent-Prothese eingesetzt werden kann. Weiterhin betrifft die Erfindung ein Kathetersystem zum Einführen einer solchen Gefäßstütze.

Bei der Behandlung von Gefäßerkrankungen, wie beispielsweise Aneurysmen ist es bekannt das erkrankte Gewebe mit einer Gefäßstütze zu stärken, oder durch eine Prothese zu ersetzen, um ein Reißen der Gefäßwand zu vermeiden. Dies ist insbesondere bei einer Erkrankung der Aorta von besonderer Bedeutung, da das Reißen der Aortenwand zu schweren inneren Blutungen und dadurch zum Tod führen kann.

Durch das Einbringen einer Prothese kann beispielsweise auch eine Dissektion des Gefäßes, insbesondere des Aortenbogens, behandelt werden. Diese Prothesen werden in der Regel durch chirurgische Verfahren eingebracht. Hierzu wird durch einen Schnitt im Bereich des Brustbeins der Brustkorb geöffnet und der Zugriff auf das erkrankte Gefäß ermöglicht. Dieser Eingriff bedeutet insbesondere aufgrund der Dauer des invasiven Eingriffs, die aufgrund der Komplexität des Verfahrens mehrere Stunden betragen kann, eine große Belastung für den Patienten. Nachdem das erkrankte Gewebe an dem Aorten-Bogen entfernt und durch eine Prothese ersetzt wurde und diese Prothese mit dem noch gesunden Gewebe vernäht wurde, gilt die Behandlung als abgeschlossen. Es hat sich allerdings gezeigt, dass obwohl das erkrankte Gewebe entfernt wurde, sich Erkrankungen, wie Dissektionen als Spätfolgen der ursprünglichen Erkrankung an dem Gewebe, das mit der Prothese vernäht wurde, fortsetzen und sich über die descendente Aorta ausbreiten können. Bei Erreichen eines gewissen Krankheitsstadiums muss dann gegebenenfalls ein erneuter Eingriff durchgeführt werden.

Das Problem, das der vorliegenden Erfindung zugrunde liegt, ist eine Vorrichtung zu schaffen, die es ermöglicht thorakale Aneurysmen und/oder Dissektionen der Aorta Descendenz in der gleichen Operation mit der Rekonstruktion der Aorta Ascendenz zu behandeln. Diese Vorrichtung soll es auch ermöglichen präventiv Spätfolgen einer Gefäßerkrankung, wie einer Dissektion oder eines Aneurysma, insbesondere des Aortenbogens, behandeln zu können, ohne bei dem ersten Eingriff noch gesundes Gewebe entfernen zu müssen. Weiterhin soll die Vorrichtung leicht herzustellen und einzusetzen sein.

Der Erfindung liegt die Erkenntnis zugrunde, dass dieses Problem ideal gelöst werden kann durch eine Gefäßstütze die nur an einem ihrer Enden an dem Gefäß permanent befestigt werden muss und an deren anderen Ende durch Kraftschluss mit der Innenseite des Gefäßes verbunden werden kann.

Die Aufgabe wird erfindungsgemäß gelöst durch eine Gefäßstütze, die einen im wesentlichen zylindrischen Schlauch aus flexiblem Material, der über seine Länge mindestens zwei aneinander angrenzende Bereiche aufweist, und mindestens ein Federelement, das im entspannten Zustand den Schlauch zumindest in dem Bereich in dem dieses vorgesehen ist, in einem geöffneten Zustand hält und das ein radiales Zusammendrücken des Schlauches zulässt, umfasst, dadurch gekennzeichnet, dass das mindestens eine Federelement in einem der Bereiche des Schlauches vorgesehen ist und der an diesen Bereich angrenzende andere Bereich frei von Federelementen ist, wobei der Bereich, in dem kein Federelement vorgesehen ist, sich bis zu einem der axialen Enden des Schlauches erstreckt.

Der Bereich des Schlauches, in dem keine Federelemente vorgesehen sind und der sich bis an eines der axialen Enden des Schlauches erstreckt, wird im folgenden auch als "freier Bereich" bezeichnet. Der Bereich, in dem mindestens ein Federelement vorgesehen ist, wird im folgenden auch als "federnder Bereich" bezeichnet.

Durch diese Ausgestaltung der Gefäßstütze kann das eine Ende des Schlauches, an dem kein Federelement vorgesehen ist, mit einem Gefäß oder einer Prothese vernäht werden und das andere Ende kann sich mittels des mindestens einen Federelementes an der Innenseite des noch gesunden Gefäßes abstützen. Auf diese Art kann beispielsweise der thorakale Aortenbogen bis in die descendente Aorta mit der Gefäßstütze von Innen unterstützt werden und dieser Bereich der Aorta braucht bei dem chirurgischen Eingriff zum Ersatz des Aortenbogens daher nicht entfernt zu werden.

Darüber hinaus weist die erfindungsgemäße Ausgestaltung der Gefäßstütze auch den Vorteil auf, dass diese leicht in das Gefäß eingeführt werden kann und zuverlässig an der eingebrachten Position verbleibt. Dies wird zum einen durch das mindestens eine Federelement und zum anderen durch die Möglichkeit des Vernähens eines der beiden Enden mit dem Gefäß oder der Aorten-Bogen-Prothese sichergestellt. Zum Einführen in das Gefäß kann darüber hinaus der Bereich des Schlauches, der frei von Federelementen ist in den anderen mit Federelementen versehenen Bereich hineingestülpt bzw. -gesteckt werden. Dies ist nur möglich, da dieser Bereich keine Federelemente aufweist und daher eine hohe Flexibilität aufweist. Durch das Hineinstecken des einen Bereiches in den anderen wird die Gesamtlänge, die die Gefäßstütze beim Einführen in das Gefäß aufweist deutlich verringert und die Platzierung der Gefäßstütze dadurch vereinfacht.

Schließlich kann sich über den Bereich, der frei von Federelementen ist ein eventuell vorliegender leichter Unterschied in dem Durchmesser der Prothese bzw. des Gefäßes, an dem die Gefäßstütze befestigt wird und dem Durchmesser, den der Schlauch durch die Federelemente annimmt ausgeglichen werden, ohne, dass ein Reißen befürchtet werden muss.

Das Vorsehen des freien Bereiches, der sich bis zu einem axialen Ende des Schlauches erstreckt, bringt auch den weitern Vorteil mit sich, dass die Platzierung der Gefäßstütze in dem Gefäß nicht mit all zu großer Präzision durchgeführt werden muss. Eine durch leichte Fehlplatzierung des federnder Bereiches verursachte, überstehende Länge des freien Bereiches, kann durch Abschneiden nach dem Einführen der Gefäßstütze in das Gefäß entfernt werden. Dadurch vereinfacht sich das Einführen und Positionieren der Gefäßstütze insgesamt und die Eingriffsdauer kann verringert werden.

Vorzugsweise erstreckt sich der Bereich, der frei von Federelementen ist, von einem Ende des Schlauches über mindestens ein Drittel der Länge des Schlauches. Unter Länge des Schlauches wird hier die Länge verstanden, die dieser beim Einführen in das Gefäß aufweist, d.h. bevor dieser eventuell im eingeführten Zustand abgeschnitten wird. Besonders bevorzugt kann sich dieser Bereich sogar über mindestens die Hälfte der Länge des Schlauches erstrecken. Durch diese Aufteilung des Schlauches in den Bereich mit und den Bereich ohne Federelemente in den bevorzugten Längen, kann zum einen die Länge der Gefäßstütze beim Einführen durch Hineinstülpen des freien Bereichs in den federnden Bereich minimiert werden, wobei dennoch gleichzeitig ein ausreichender Halt des mit Federelementen versehenen Bereichs an der Innenseite des Gefäßes sicher gestellt werden kann.

Das mindestens eine Federelement erstreckt sich vorzugsweise um den gesamten Umfang des Schlauches. Dadurch kann ein gleichmäßiges Zusammendrücken und Auseinanderziehen des Schlauches in radialer Richtung erzielt werden.

Vorzugsweise ist das mindestens eine Federelement an der Außenseite des Schlauches befestigt. Durch diese Anordnung der Federelemente können mehrere Vorteile erzielt werden. Zum einen kann bei geeigneter Wahl des Materials der Federelemente erzielt werden, dass diese in das Gewebe einwachsen können, wodurch der Halt der Gefäßstütze noch verbessert wird. Weiterhin können Turbulenzen, die bei im Inneren des Schlauches liegenden Federelementen auftreten könnten, vermieden werden. Schließlich wird durch die Federelemente keine Verengung des Lumens der Gefäßstütze erzeugt bzw. diese minimiert. Die Federelemente, die an der Außenseite des Schlauches befestigt sind, drücken sich an und gegebenenfalls auch leicht in die Gefäßwand, wodurch der Schlauch an diesen Stellen radial nach außen gezogen wird, aber auf jeden Fall eine Verengung an diesen Stellen ausgeschlossen werden kann. Somit wird ein im wesentlichen gleichmäßiger Durchschnitt des Schlauches über dessen Länge auch in der unmittelbaren Nähe der Position, an der das mindestens eine Federelement befestigt ist, gewährleistet. Weiterhin kann, wenn die Federelemente an der Außenseite des Schlauches angebracht sind, der freie Bereich des Schlauches zum Einführen der Gefäßstütze in das Gefäß in den federnden Bereich hineingestülpt werden und aus diesen herausgezogen werden, ohne, dass ein Verhaken des freien Bereiches mit den Federelementen befürchtet werden muss.

Besonders bevorzugt ist das mindestens eine Federelement an der Außenseite des Schlauches angenäht. Diese Art der Befestigung kann zum einen ein Verrutschen der Federelemente an dem Schlauch vermeiden und ist zum anderen gesundheitlich unbedenklich. Darüber hinaus ist es durch diese Art der Befestigung weiterhin möglich, dass die Federelemente von dem Gefäßgewebe aufgenommen werden und gegebenenfalls mit diesem verwachsen können.

In einer Ausführungsform stellt das mindestens eine Federelement einen Federring dar. Im Sinne der Erfindung kann das mindestens eine Federelement verschiedene Formen aufweisen und aus verschiedenen Materialien hergestellt sein. Es ist allerdings bevorzugt, dass das Element eine Federwirkung aufweist, durch die dieses den Schlauch in dem geöffneten Zustand halten kann. Daher ist ein Federring bevorzugt. Dieser umgibt vorzugsweise in dem entspannten Zustand den Umfang des Schlauches und kann durch Druck auf die Längsachse des Schlauches zu bewegt, d.h. zusammengedrückt, werden. Durch Beseitigung des Druckes kann sich der Federring ausweiten und den Schlauch in seiner im wesentlichen zylindrischen Form halten.

In einer bevorzugten Ausführungsform erstreckt sich der Bereich, in dem mindestens ein Federelement vorgesehen ist, bis an das axiale Ende, das dem Ende, an dem der Bereich der frei von Federelementen ist, endet, gegenüberliegt. Dies bedeutet, dass an dem Ende des Schlauches, der dem freien Bereich gegenüberliegt, ein Federelement vorgesehen ist. Dadurch kann verhindert werden, dass über das letzte Federelement an diesem Ende des Schlauches überstehendes flexibles Material des Schlauches den Blutstrom behindert.

Die Anzahl der Federelemente, die in einem Bereich des Schlauches vorgesehen sind, kann beispielsweise mindestens drei, vorzugsweise mindestens 4 und besonders bevorzugt mindestens 5 betragen.

Die erfindungsgemäße Gefäßstütze kann beispielsweise eine Aorten-Stent-Prothese darstellen. Bei diesem Einsatz der erfindungsgemäßen Gefäßstütze können die Vorteile der Gefäßstütze ideal genutzt werden, da nach dem Ersatz des Aortenbogens durch eine Prothese der Blutdruck, der auf den thorokalen Aortenbogen und den oberen Bereich der descenden Aorta wirkt erhöht ist. Hier kann die erfindungsgemäße Gefäßstütze also ideal als Präventivmittel oder Hilfsmittel bei schon erkrankten Gefäßabschnitten wirken. Insbesondere kann bei einem solchen Einsatz der Vorteil der erfindungsgemäßen Gefäßstütze, dass diese eine Kombination aus einer Prothese und einem Gefäßstent bildet, genutzt werden, da die Gefäßstütze zu einen, wie eine Prothese an das Gewebe genäht werden kann und wie ein Stent durch Kraftschluss mit der Gefäßwand zusammenwirken kann.

Die vorliegende Erfindung betrifft weiterhin ein Kathetersystem zum Einführen einer erfindungsgemäßen Gefäßstütze, wobei das Kathetersystem einen Katheter, der zumindest ein Lumen aufweist, in das die Gefäßstütze aufgenommen werden kann, und eine in dem ersten Lumen angeordnete längsbewegliche Vorrichtung umfasst, die an ihrem distalen Ende eine Befestigungsvorrichtung aufweist, die mit dem Ende des Schlauches an dem der Bereich, der frei von Federelementen ist, angeordnet ist, verbunden werden kann. Diese Befestigungsvorrichtung kann insbesondere, wenn der freie Bereich der Gefäßprothese in den federnden Bereich eingestülpt ist, zum Herausziehen des freien Bereiches dienen. Vorzugsweise stellt die längsbewegliche Vorrichtung in dem Kathetersystem daher eine Zugvorrichtung dar.

Das Kathetersystem kann erfindungsgemäß einen Ballonkatheter umfassen. Vorzugsweise ist dann in der Befestigungsvorrichtung ein Durchlass vorgesehen, durch den der Ballon im entspannten Zustand geführt werden kann. Diese Ausgestaltung des Kathetersystems erlaubt es, dass der Ballon im entspannten Zustand in einer Position gehalten wird, die von der Position der Gefäßstütze beabstandet ist, und dass der Ballon nach dem Einbringen der Gefäßstütze in das Gefäß in eine Position gebracht werden kann, in der er sich innerhalb der Gefäßstütze befindet. Wird der Ballon dann in der letztgenannten Position dilatiert, so kann er die Gefäßstütze an die Gefäßwand andrücken und, falls notwendig, auch das Gefäß aufweiten. Beim Einführen der Gefäßstütze behindert der Ballon, aufgrund seiner beabstandeten Position, nicht.

Die Erfindung wird im Folgenden anhand der beiliegenden Zeichnungen, die sich auf mögliche Ausführungsbeispiele der Erfindung beziehen, beschrieben. Es zeigen:
Figur 1: eine schematische Darstellung einer erfindungsgemäßen Gefäßstütze;
Figur 2: eine schematische Darstellung einer erfindungsgemäßen Gefäßstütze im eingestülpten Zustand;
Figur 3: eine schematische Darstellung einer erfindungsgemäßen Gefäßstütze im zweifach eingestülpten Zustand;
Figuren 4a- 4c: schematische Darstellungen einer erfindungsgemäßen Gefäßstütze in einem erfindungsgemäßen Kathetersystem, in verschiedenen Situationen beim Einführen in ein Gefäß;
Figur 5: eine schematische Darstellung einer erfindungsgemäßen Gefäßstütze im, in ein Gefäß eingesetzten Zustand.

Die Darstellungen sind nicht maßstabsgetreu.

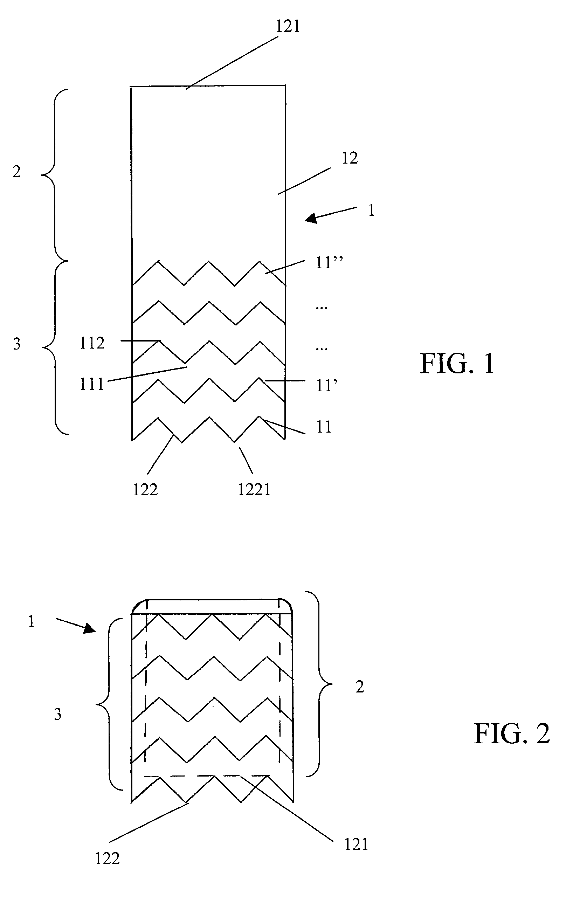

In Figur 1 ist eine schematische Darstellung einer erfindungsgemäßen Gefäßstütze 1 gezeigt. Der Körper der Gefäßstütze 1 wird durch einen Schlauch 12 aus flexiblem Material gebildet. Dieser Schlauch 12 weist ein distales Ende 121 und ein proximales Ende 122 auf. In der dargestellten Ausführungsform wird das proximale Ende 122 durch Spitzen 1221 des Schlauches 12 gebildet. Die Spitzen 1221 sind gleichmäßig über den Umfang des proximalen Endes 122 des Schlauches 12 verteilt angeordnet. Das distale Ende 121 stellt einen glatten Abschluss dar.

An dem proximalen Ende 122 ist entlang dem Verlauf der Spitzen 1221 ein Federelement 11 geführt und dort befestigt. Vorzugsweise ist dieses Federelement 11 an der Außenseite des Schlauches 12 angenäht. Es liegt aber auch im Rahmen der Erfindung, dass dieses Federelement 11 an der proximalen Kante des Schlauches 12 entlang der Form der Spitzen 1221 befestig ist. In Längsrichtung des Schlauches 12 in Richtung auf das distale Ende 121 des Schlauches 12 zu, sind in der dargestellten Ausführungsform weitere Federelemente 11' bis 11" in gleichmäßigen Abständen an dem Schlauch 12 vorgesehen. Diese Federelemente 11' bis 11" weisen die gleiche Form, wie das Federelement 11 auf und bilden somit geschlossenen Ringe um den Schlauch, die in die Längsrichtung des Schlauches eine Zick-Zack-Form aufweisen. Die Federelemente 11, 11' bis 11" sind so angeordnet, dass deren Täler 111 und Höhen 112 auf einer Linie parallel zu der Längsachse des Schlauches mit den jeweiligen Tälern und Höhen der weiteren Federelemente liegen.

Dadurch werden entlang des Schlauches 12 zwei Bereiche 2 und 3 gebildet, die über die Länge des Schlauches 12 aneinander angrenzend angeordnet sind. Der Bereich 3 erstreckt sich von dem proximalen Ende 122 des Schlauches 12 bis zu den Höhen 112 des von diesem am weitesten entfernten Federelementes, d.h. des oberen Federelementes 11". An diesen Bereich 3, der im folgenden auch als federnder Bereich bezeichnet wird, schließt sich der Bereich 2 an, der frei von Federelementen ist und im folgenden auch als freier Bereich 2 des Schlauches 12 bezeichnet wird. Der Bereich 2 endet an dem distalen Ende 121 der Gefäßstütze 1. In dem freien Bereich 2 sind keinerlei Federelemente oder andere Verstärkungselemente vorgesehen. Dieser Bereich 2 wird ausschließlich durch das Schlauchmaterial gebildet.

In Figur 2 ist die Gefäßstütze 1 aus Figur 1 schematisch in einem eingestülpten Zustand gezeigt. Hierbei ist der Bereich 2 in den Bereich 3 hineingestülpt. In diesem Zustand kann die Gefäßstütze in ein Einführsystem, wie einen Katheter eingebracht werden.

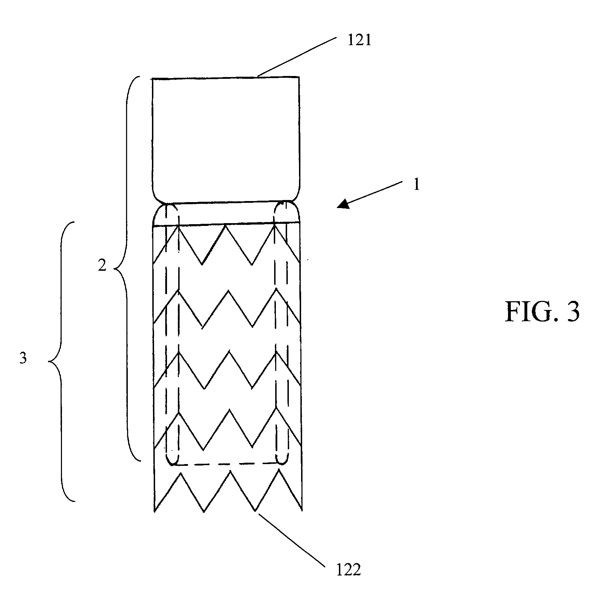

In Figur 3 ist eine weitere Möglichkeit des Einstülpens des freien Bereiches 2 gezeigt. In diesem Fall ist der freie Bereich 2 doppelt gefaltet. Hierdurch wird das distale Ende 121 der Gefäßstütze 1 von der Seite an der dieses sich nach dem Entfalten befindet aus zugänglich. Ein Herausziehen des freien Bereiches 2 aus dem Bereich 3 wird daher erleichtert. Die Länge über die sich der freie Bereich 2 im zusammengefalteten Zustand in den Bereich 3 erstreckt, hängt von dessen Gesamtlänge ab. Vorzugsweise wird der freie Bereich 2 fast vollständig in dem Bereich 3 aufgenommen und nur ein geringer Betrag steht über den Bereich 3 hinaus. Um den Durchmesser einer in einer solchen Weise eingestülpten Gefäßstütze gering halten zu können, was für das Einführen der Gefäßstütze in das Gefäß von Bedeutung ist, ist es wesentlich, dass der freie Bereich 2 keine Verstärkungselemente oder andere Federelemente aufweist, sondern lediglich aus dem Schlauchmaterial besteht.

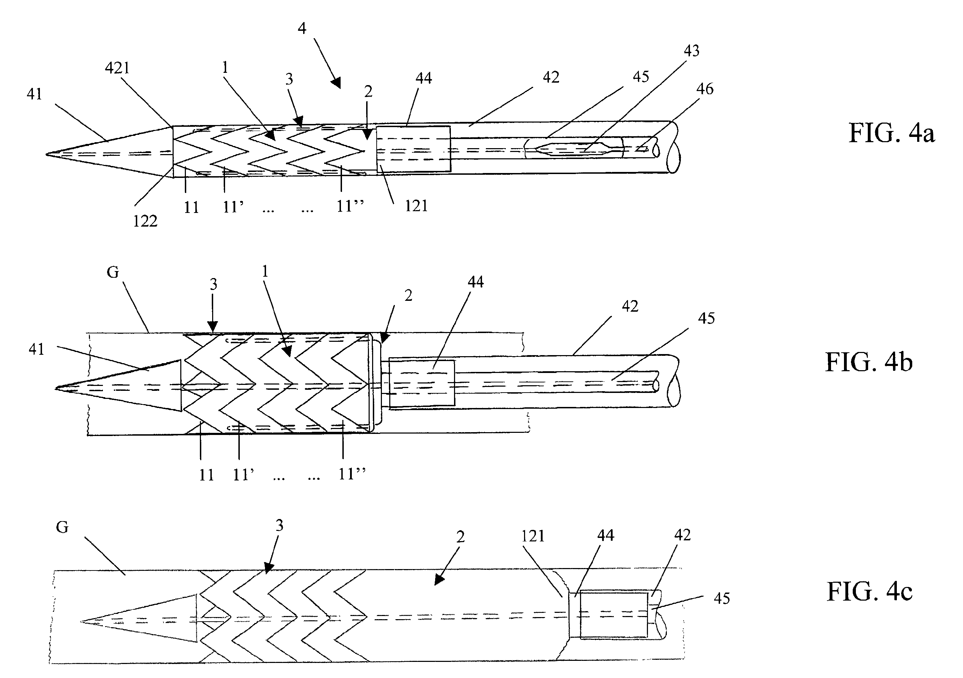

In Figuren 4a bis 4c ist ein erfindungsgemäßes Kathetersystem 4 dargestellt, in dem eine erfindungsgemäße Gefäßstütze 1 aufgenommen ist. Figur 4a zeigt das Kathetersystem 4 mit der darin aufgenommenen Gefäßstütze 1 in dem Zustand, in dem dieses in das Gefäß, wie beispielweise die Aorta, eingeführt werden kann.

Das Kathetersystem 4 weist in der dargestellten Ausführungsform eine im wesentlichen zylindrische Hülse 42 auf, die an einem Ende 421 durch eine Führungsspitze 41 verschlossen ist. Die Führungsspitze 41 ist über einen Führungsdraht (nicht dargestellt) beweglich. Dieser Führungsdraht wird in einem Führungsdrahtlumen 46, das sich über die gesamte Länge der Hülse 42 erstreckt, geführt.

In der Hülse 42 ist eine Befestigungsvorrichtung 44 für die Gefäßstütze 1 geführt. Diese Befestigungsvorrichtung 44 stellt beispielsweise einen Zylinder dar. Dieser weist in dessen radialer Mitte einen Durchlass für den Führungsdraht 43 auf und ist an dem Ende, dass dem distalen Ende des Katheters abgewandt ist, mit einer Führungshülse 45 verbunden, die in der Hülse 42 geführt ist.

Im Bereich zwischen der Befestigungsvorrichtung und dem proximalen Ende der Führungshülse 45 ist ein Ballon 43 dargestellt. Dieser Ballon 43 kann über einen Ballonkatheter (nicht dargestellt) verschoben und dilatiert werden. Es kann somit ein Ballonkatheter in das Kathetersystem integriert werden, der auf dem Führungsdrahtlumen 46 gleiten kann und in der Führungshülse 45 läuft. Vorzugsweise befindet sich der Ballon 43 des Ballonkatheters zum Einführen des Kathetersystems 4 in das Gefäß in der in Figur 4a dargestellten Position, d.h. von der Führungsspitze 41 aus betrachtet hinter der Befestigungsvorrichtung.

An den proximalen Enden der Hülse, der Führungshülse, des Ballonkatheters und des Führungsdrahtes, können Betätigungsvorrichtungen vorgesehen sein, die das Bewegen dieser Komponenten unabhängig voneinander erlauben. Diese sind in Figur 4 nicht gezeigt.

Die Gefäßstütze 1 ist in dem Kathetersystem so angeordnet, dass deren proximales Ende 122 nahe an dem distalen Ende 421 der Hülse 42 liegt. In dem sich an das proximale Ende 122 der Gefäßstütze 1 anschließenden Bereich 3, in dem die Federelemente 11, 11' bis 11" vorgesehen sind, ist der freie Bereich 2 aufgenommen. In der dargestellten Ausführungsform ist der freie Bereich 2 doppelt gefaltet in den Bereich 3 eingeführt. Der freie Bereich 2 verläuft daher beginnend von dem letzten Federelement 11" zunächst in die Richtung des proximalen Endes 122 der Gefäßstütze 1 und ist dann so umgeklappt, dass dieser in die Richtung, die dem proximalen Ende 122 der Gefäßstütze 1 entgegen gesetzt ist, weiter verläuft. An dem distalen Ende 121, das den freien Bereich 2 abschließt, ist die Gefäßstütze 1 mit der Befestigungsvorrichtung 44 verbunden. Diese Verbindung kann durch Ankleben, Annähen, Klemmen oder durch andere Verbindungsmechanismen, wie Verbindungsmagneten erfolgen. Vorzugsweise wird der Schlauch 12 an der Befestigungsvorrichtung 44 mit seinem distalen Ende 121 angenäht.

In Figur 4b wird der Zustand gezeigt, den das Kathetersystem 4 und die Gefäßstütze 1 annehmen, wenn die Gefäßstütze 1 aus dem Kathetersystem 4 entlassen werden soll. Hierbei wird die Hülse 42 entlang ihrer Längsachse zurückgezogen, d.h. in Richtung ihres proximalen Endes bewegt. Dadurch entfernt sich die Führungsspitze 41 von der distalen Öffnung der Hülse 42. Die Führungshülse 45 und die damit verbundene Befestigungsvorrichtung 44 und die Gefäßstütze 1 verändern ihre Position dabei in Längsrichtung nicht. Allerdings federn die Federelement 11, 11' bis 11", die durch die Hülse 42 auf einen kleineren Durchmesser reduziert worden waren, auf und legen sich an die Gefäßwand (G) an. Es ist auch möglich, dass die Befestigungsvorrichtung 44 in ihrem Durchmesser so bemessen ist, dass diese unmittelbar an der Innenseite der Hülse 42 anliegt und beim Zurückziehen der Hülse 42 als Gegenhalt des federnden Bereiches 3 dient oder zum Herausschieben des federnden Bereiches 3 aus der Hülse 42 verwendet werden kann. Wenn der gesamte federnde Bereich 3 aus der Hülse 42 entlassen ist, kann der in diesem federnden Bereich 3 aufgenommene freie Bereich 2 aus dem federnden Bereich herausgezogen werden.

Dies ist in Figur 4c angedeutet. Hierzu wird die Führungshülse 45, die über die Befestigungsvorrichtung 44 mit dem distalen Ende 121 der Gefäßstütze 1 verbunden ist, in Richtung auf das proximale Ende der Hülse 42 hin bewegt. Dadurch wird der freie Bereich 2 aus dem federnden Bereich 3 herausgezogen. Es ist auch möglich, dass zusammen mit der Führungshülse 45 die Hülse 42 in die Richtung auf deren proximales Ende hin bewegt wird.

Hat die Gefäßstütze 1 die in Figur 4c gezeigte gestreckte Form angenommen ist der Einführungsvorgang beendet. Sofern ein Ballon 43 in dem Kathetersystem vorgesehen ist, wird der Ballon 43 nun bis zum proximalen Ende 122 vorgeschoben und dilatiert bzw. expandiert. Beginnend von diesem Ende 122 wird jeder Bereich des Schlauches 12, beinhaltend die Bereiche 2 und 3, mittels des Ballons 43 an die Gefäßwand G anmodelliert. Die Gefäßstütze 1 kann dann von der Befestigungsvorrichtung 44 getrennt und das Kathetersystem aus dem Gefäß und der Gefäßstütze 1 entfernt werden. Je nach dem gewählten Verbindungsmechanismus zwischen der Befestigungsvorrichtung 44 und der Gefäßstütze 1 kann die Verbindung durch Entfernen der Verbindungsmittel, wie Klebstoff, gelöst werden, oder die Gefäßstütze 1 kann in der Nähe der Befestigungsvorrichtung 44 abgeschnitten werden. Durch diese letzte Variante kann sicher gestellt werden, dass die Gefäßstütze 1, unabhängig von der Platzierung des federnden Bereiches 3, die korrekte Länge aufweist.

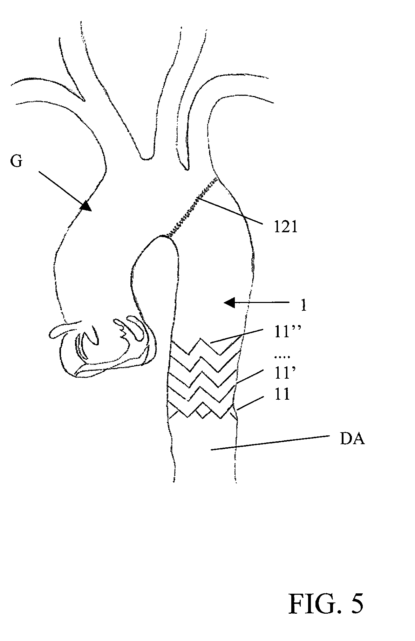

In Figur 5 ist schließlich ein in ein Gefäß (hier die Aorta) eingesetzte Gefäßstütze 1 gemäß der Erfindung gezeigt. Das distale Ende 121 der Gefäßstütze 1 ist mit der Gefäßwand (G) im Bereich des Aortenbogens vernäht. Das proximale Ende 122 der Gefäßstütze 1 liegt mit den daran vorgesehenen Federelementen 11, 11' bis 11" an der Innenseite der decendenten Aorta (DA) an.

Die oben beschriebenen Ausführungsformen der erfindungsgemäßen Gefäßstütze und des erfindungsgemäßen Kathetersystems sind lediglich Beispiele der Verwirklichung der Erfindung. Die Erfindung ist aber nicht auf diese beschränkt.

So kann die Anzahl der Federelementen je nach Verwendung der Gefäßprothese variiert werden. Auch der Abstand zwischen den einzelnen Federelementen kann je nach Bedarf festgelegt werden. So können beispielsweise bis zu 15 Federn in gleichmäßigen Anständen von 5 bis mm vorgesehen sein. Auch die Form der Federelemente ist nicht auf die beschriebene Zick-Zack-Form beschränkt. Solange das Federelement zum einen eine Verringerung des Durchmessers der Gefäßstütze auf den Innendurchmesser der Hülse eines Katheters erlaubt und zum anderen den Schlauch der Gefäßprothese im entspannten zustand auf die gewünschte Größe aufweiten kann, können auch andere Federelemente eingesetzt werden. Diese können beispielsweise Federringe sein, die in axialer Richtung eine Wellen- oder Kronenform aufweisen. Weiterhin ist es auch möglich zwischen den einzelnen Federelementen Verbindungselemente vorzusehen, die beispielsweise einen Draht, der sich in axialer Richtung zwischen den einzelnen Federelementen erstreckt, darstellen können. Hierdurch kann die Länge des federnden Bereiches konstant gehalten werden und diesem Bereich eine Stabilität gegen Verdrehen gegeben werden.

Die Lange der Gefäßprothese kann ebenfalls variieren. Beispielsweise kann die Gefäßstütze mit einer Länge von 25- 30cm hergestellt werden. Die Länge der Gefäßstütze im eingesetzten Zustand kann dann, wie oben beschrieben durch Abschneiden eingestellt werden. Die Länge des federnden Bereiches beträgt beispielsweise 5 bis 15 cm, über den dann die gewünschte Anzahl Federelemente verteilt ist.

Als Materialien für die Gefäßstütze und das Kathetersystem können sämtliche in der Medizintechnik für die jeweiligen Teile üblichen Materialien verwendet werden. Der Schlauch kann aus einem Polyestermaterial, insbesondere Polyesterfaser-Material, wie beispielsweise aus einem Dacron-Material hergestellt sein, da dieser zum einen sehr flexibel ist, sich daher leicht in den federnden Bereich einstülpen lässt, zum anderen aber gut vernäht werden kann und sich auch leicht schneiden lässt. Als Material für die Federelemente kann beispielsweise Nitinol-Draht oder ein Draht aus rostfreiem Stahl (stainless steel) gewählt werden.

Auch die Art der Verbindung des distalen Endes 121 des Schlauches mit dem Kathetersystem, insbesondere mit der Befestigungsvorrichtung 44, kann frei gewählt werden. Es ist beispielsweise möglich den Schlauch durch Klemmen, Verkleben, Annähen, Stecken oder mittels eines Magnetrings mit der Befestigungsvorrichtung zu verbinden.

Die Federelemente, die erfindungsgemäß vorzugsweise an der Außenseite des Schlauches befestigt werden, können dort beispielsweise angeklebt, in das Schlauchmaterial eingearbeitet oder aber an den Schlauch angenäht werden. Hierbei können die Federelemente über ihre gesamte Länge mit dem Schlauch durch eine durchgehende Naht verbunden werden. Es ist aber auch möglich die Federelemente lediglich durch einzelne Stiche in größeren Abständen über deren Länge mit dem Schlauch zu vernähen.

Die Anbringung der Federelemente erfolgt bevorzugt an der Außenseite des Schlauches. Es liegt aber auch im Rahmen der Erfindung die Federelemente an der Innenseite des Schlauches vorzusehen, oder diese in das Schlauchmaterial zu integrieren. Für eine Befestigung der Federelemente an der Innenseite können die gleichen Befestigungsarten gewählt werden, wie diese für die Anbringung an der Außenseite oben beschrieben sind.

Es ist auch möglich das proximale Ende 122 des Schlauches so zu gestallten, dass das Schlauchmaterial einen glatten Anschluss aufweist, d.h. keine Spitzen aufweist. In diesem Fall können sowohl zick-zack-geformte Federelemente, als auch ebene ringförmige Federelemente verwendet werden.

Obwohl das Vorsehen von nur zwei Bereichen, einem federnden und einem freien, die sich jeweils bis zu den Enden des Schlauches erstrecken, bevorzugst ist, können beispielsweise auch drei Bereiche vorgesehen sind, wobei hierbei zwei freie Bereiche sich von den beiden Enden erstrecken und einen federnden Bereich zwischen sich einschließen.

Die Hülse des Katheters kann einen über ihre Länge gleichbleibenden Durchmesser aufweisen. Die Hülse kann aber auch an ihrem distalen Ende einen Bereich aufweisen, dessen Durchmesser im Vergleich zu dem Durchmesser der restlichen Länge der Hülse größer ist. In diesen Bereich kann die Gefäßstütze ideal aufgenommen werden.

Der Führungsdraht, der in dem Kathetersystem verwendet wird kann auch hohl, d.h. in Form eines Schlauches ausgebildet sein.

Mit der erfindungsgemäßen Gefäßstütze und dem erfindungsgemäßen Kathetersystem kann somit eine Vorrichtung geliefert werden, die sich leicht in zu behandelnde Gefäße einführen lässt, in diesen Gefäßen sicher befestigt werden kann und so die präventive Behandlung von Gefäßgewebeerkrankungen, wie der Dissektion ermöglicht.

## Patentansprüche

1. Gefäßstütze (1), die im wesentlichen zylindrischen Schlauch (12) aus flexiblem Material, der über seine Länge mindestens zwei aneinander angrenzende Bereiche (2, 3) aufweist, und mindestens ein Federelement (11, 11', 11"), das im entspannten Zustand den Schlauch (12) zumindest in dem Bereich (3) in dem dieses vorgesehen ist, in einem geöffneten Zustand hält und das ein radiales Zusammendrücken des Schlauches (12) zulässt, umfasst, **dadurch gekennzeichnet, dass** das mindestens eine Federelement (11, 11', 11") in einem der Bereiche (3) des Schlauches vorgesehen ist und der an diesen Bereich (3) angrenzende andere Bereich (2) frei von Federelementen (11, 11', 11") ist, wobei der Bereich (2), in dem kein Federelement (11, 11', 11") vorgesehen ist, sich bis zu einem der axialen Enden (121) des Schlauches (12) erstreckt.

2. Gefäßstütze gemäß Anspruch 1, **dadurch gekennzeichnet, dass** der Bereich (2), der frei von Federelementen ist, sich von einem Ende (121) des Schlauches (12) über mindestens ein Drittel der Länge des Schlauches (12) erstreckt.

3. Gefäßstütze nach einem der Ansprüche 1 oder 2, **dadurch gekennzeichnet, dass** sich das mindestens eine Federelement (11, 11', 11") um den gesamten Umfang des Schlauches (12) erstreckt.

4. Gefäßstütze gemäß Anspruch 1 oder 2, **dadurch gekennzeichnet, dass** das mindestens eine Federelement (11, 11', 11") an der Außenseite des Schlauches (12) befestigt ist.

5. Gefäßstütze gemäß einem der Ansprüche 1 bis 3, **dadurch gekennzeichnet, dass** das mindestens eine Federelement (11, 11', 11") an der Außenseite des Schlauches (12) angenäht ist.

6. Gefäßstütze gemäß einem der Ansprüche 1 bis 4, **dadurch gekennzeichnet, dass** die Federelemente (11, 11', 11") Federringe darstellen.

7. Gefäßstütze gemäß einem der Ansprüche 1 bis 5, **dadurch gekennzeichnet, dass** sich der Bereich (3), in dem mindestens ein Federelement (11, 11', 11") vorgesehen ist, bis an das axiale Ende (122) erstreckt, das dem Ende (121) gegenüberliegt, an dem der Bereich (2) der frei von Federelementen (11, 11', 11") ist, endet.

8. Gefäßstütze gemäß einem der vorstehenden Ansprüche, **dadurch gekennzeichnet, dass** mindestens fünf Federelemente (11, 11', 11") vorgesehen sind.

9. Gefäßstütze gemäß einem der vorstehenden Ansprüche, **dadurch gekennzeichnet, dass** diese eine Aorten-Stent-Prothese darstellt.

10. Kathetersystem zum Einführen einer Gefäßstütze gemäß einem der Ansprüche 1 bis 8, **dadurch gekennzeichnet, dass** das Kathetersystem (4) einen Katheter, der zumindest ein Lumen (42) aufweist, in das die Gefäßstütze (1) aufgenommen werden kann, und eine in dem ersten Lumen (42) angeordnete längsbewegliche Vorrichtung (45) umfasst, die an ihrem distalen Ende eine Befestigungsvorrichtung (44) aufweist, die mit dem Ende des Schlauches (12) an dem der Bereich (2), der frei von Federelementen (11, 11', 11") ist, angeordnet ist, verbunden werden kann.

11. Kathetersystem nach Anspruch 10, **dadurch gekennzeichnet, dass** die längsbewegliche Vorrichtung (45) eine Zugvorrichtung darstellt.

12. Kathetersystem nach Anspruch 10 oder 11, **dadurch gekennzeichnet, dass** dieses einen Ballonkatheter umfasst.

13. Kathetersystem nach Anspruch 12, **dadurch gekennzeichnet, dass** die Befestigungsvorrichtung (44) einen Durchlass aufweist, durch den der Ballon (43) im entspannten Zustand geführt werden kann.
